# EUROPEAN PATENT SPECIFICATION

(11) **EP 2 351 530 B1**
(45) Date of publication and mention of the grant of the patent: **23.01.2013**
(21) Application number: 10000859.8
(22) Date of filing: 28.01.2010
(51) Int. Cl.: A61B 17/22, A61N 7/00

(54) **Handheld shockwave apparatus having a pressing device**
Tragbare Schockwellenapparatur mit einer Pressvorrichtung
Appareil portable d'ondes de choc doté d'un dispositif de pression

(43) Date of publication of application: 03.08.2011
(73) Proprietor: STORZ MEDICAL AG, 8274 Tägerwilen (CH)
(72) Inventor: Del Giglio,Antonio, Dr., 37100 Verona (IT); Marlinghaus, Ernst H., CH-8598 Bottighofen (CH)
(74) Representative: Szynka, Dirk

(56) References cited:
- WO-A2-2009/050719
- DE-U1-202007 007 921
- US-A1- 2007 239 082
- US-A1- 2009 171 255
- US-A1- 2009 221 938

## Description

The present invention relates to a handheld shockwave apparatus having a pressing device.

Shock waves, i.e. mechanical waves sometimes also named "acoustic" or "pressure waves", are presently used in different ways for therapeutic treatment. Shock wave lithotripsy is especially important and has been the starting point of the development in a historical sense, namely the disintegration of concrements in the body, especially stones, using focused shock waves of high amplitude and steep rising edges. Besides a generation by electric discharges in water, apparatuses have been developed, which generate shockwaves electromechanically in water. Further, apparatuses are known, which generate shockwaves by a mechanical impact of an accelerated striking means onto an impact body. Shock wave apparatuses can, besides the use in lithotripsy, also be applied for other treatments of biological matters of the body, as for example the muscles. Therein, also handheld apparatuses are known, wherein an operator applies an applicator head of the apparatus by pressing it against the body, see for instance US 2007/0239082 A1 and DE 20 2007 007 921 U1.

US 2009/0221938 A1, on which the preamble of claim 1 is based, discloses a double ultrasonic treatment apparatus, wherein two ultrasound transducers are arranged symmetrically and can be moved towards each other by a linear displacement The transducers are respectively connected to plates, which have a layer with a high coefficient of friction such that skin and tissue is pushed up by this linear displacement and is brought in-between the transducers.

US 2009/0171255 A1 discloses an apparatus with piezoelectric transducers for an ultrasound treatment, wherein the transducers are arranged in two arrays, each of which is provided on a griping panel. The panels are connected to each other by a flexure hinge and can be moved away from each other by applying pressure to a spreading handle. By releasing pressure from the handle, the body part to be treated can be clamped between the gripping panels.

WO 2009/050719 A2 discloses ultrasonic transducers, which are connected to each other by a support element and can be moved with respect to each other by the support element. Likewise, the distance between the transducers can be varied and a body part to be treated can be sandwiched between the transducers.

The problem of the present invention is to provide a handheld shockwave apparatus, which shows improved properties in terms of handling, performance, and/or safety.

The problem is solved with a handheld apparatus according to claim 1.

The shock wave apparatus can be supplied by and can be connected to a basic unit, wherein this basic unit can even be permanently installed. However, the shock wave apparatus itself is a handheld unit.

The shockwave source can produce shockwaves, which propagate for example in a main propagation direction (and possibly many other directions, for example radially, so that the main propagation direction is a mean or average direction of propagation) and the pressing device is located at a certain distance from the application head in this main propagation direction. Therein, the pressing device is mechanically connected to the shockwave apparatus and can be moved towards the application head, for example in the main propagation direction, for pressing the body part against the application head in a sandwich-like manner. Therefore, the contact pressure of the application head does not only, as conventionally known, result from pressing the application head against the body and a more or less elastic response of the body itself. It is thus easy to produce sufficient contact pressure and in certain situations, for example on the abdomen, less disturbing for the patient.

Depending on the way of use, sandwiching the body part to be treated can also protect inner parts of the body, in particular the skeleton, the teeth and the organs. If for example a part of the torso is treated, the skin and the underlying tissue can be pulled slightly away from the body when being sandwiched between the application head and the pressing device. In this case, above-mentioned main propagation direction of the shockwaves, which is pointing towards the pressing device, is more a tangent with respect to the torso than penetrating the torso. In this way of use, the shockwaves can penetrate the body part to be treated in a well defined manner and only the body part to be treated. Otherwise, the energy loss of a shockwave caused by a change of impedance, which can occur for example when propagating from a region rich in water to a region rich in gas, could cause severe damage to an organ, e.g. the lung.

In addition, pressing the body part to be treated between the application head and the pressing device can have, prior or in addition to the actual shockwave, a mechanical effect on the body part in terms of a massage-like treatment. In context of a mechanical treatment of the skin and the underlying tissue prior to and during the actual shockwave, pressing the body part can further lead to a segregation of fat, as the permeability of the cells can be increased in the state of being pressed.

Preferred embodiments follow from the dependant claims and the following description. Therein, the details refer to all aspects of invention and to the different types of claims, this without being differentiated explicitly in detail.

According to the invention, the pressing device comprises a pressing plate extending approximately transversely to the above-mentioned main propagation direction of the shockwaves. Therewith, transversal extension with respect to the main propagation direction refers to the state, when the body part to be treated is sandwiched between the application head and the pressing device. Likewise, the pressing plate can be shifted, folded, or pivoted away and can have an arbitrary orientation with respect to the application head and the main propagation direction, when the body part is not sandwiched. The plate can be formed for example of metal or synthetic material, wherein also combinations are possible, as for example a plastic laminated metal. Braces can be provided additionally to increase the stiffness of the plate.

Preferably, the pressing device of the apparatus according the invention has a pressing surface with a concave shape. The pressing surface is that surface of the pressing device, which faces the application head when sandwiching the body part between application head and pressing device. Therein, the pressing surface can have a certain roughness or even show a modulation with recurring amplitude or frequency, as for example grooves or ripples. However, a smooth surface is preferred and the shape of the surface as a whole can be concave. On one hand, the concave shape of the pressing plate allows a pressing of the body part against the application head not only in one direction, but instead "focuses" the body part from several directions radially towards the application head. On the other hand, a concavely shaped pressing plate will, when acting as a reflector with a smooth surface, not diffuse the shockwaves by a diverging reflection, but instead can focus the reflected shockwaves into the body part to be treated. Focusing reflected shockwaves enables a further interaction of the reflected waves with the body part to be treated. Likewise, the effectiveness of the shockwave treatment can be increased. Therefore, a concave shape of the pressing surface is preferred, even though, a plane or convex shape would also be possible and may be preferred in certain applications as well.

According to another embodiment of the invention, the pressing device has a pressing surface which is formed of metal. The metal surface has, in comparison to the skin and the underlying tissue, a high impedance. Therefore, a metal coated pressing device or a full metal pressing device can at least partially reflect a shockwave propagating from the application head through the body part to the metal surface. On one hand, this can increase the interaction with the body part to be treated and, on the other hand, it can prevent body parts lying outside the sandwiched region, in particular on the opposite side of the pressing device, from an unintended shockwave treatment. The pressing surface could also be formed of a foamed material, to provide an absorption layer. However, a reflective layer formed of metal is preferred.

Advantageously, the pressing device comprises, in a main propagation direction of the shockwaves a layer of metal and a layer of foamed material in sequence. The sequence of materials refers to above-mentioned main propagation direction, when the body part is sandwiched between the pressing device and the application head. The sequence with respect to the main propagation direction can be different, when the pressing device is folded or pivoted away. The shock waves are, as described above, at least partially reflected by the metal surface. However, a certain part might still propagate through the pressing device and interact unintendedly with other parts of the body. Therefore, a layer of foam material can be provided in sequence to the metal, so that the change of impedance causes a further energy loss for that part of the shock waves being transmitted. Likewise, the foamed material can act as an absorption layer and can further prevent an unintended propagation of the shockwaves.

The pressing device can be a treatment head of a source of energetic radiation, e.g. a shockwave source, an ultrasonic source, an infrared source, or a microwave source. Likewise, an additional treatment device is provided, which enables a treatment of the body part in a direction opposing to the main propagation direction of the first shockwave source. With this embodiment, the body part to be treated could for example be prepared for the shockwave treatment by a heat treatment with an infrared source or the permeability of the cells could be increased with an ultrasonic or microwave treatment prior to and during the shockwave treatment, respectively. Alternatively, it is also possible to increase the shockwave density by providing two mechanically connected shockwave apparatuses, wherein the body part to be treated is sandwiched between two application heads. Therein, also shock wave apparatuses with a different type of shock wave generation can be provided, e.g. according to claims 10 and 11 and to claims 12 and 13 respectively.

The pressing device is hinged, so that it can be pressed against the body part by means of a pivoting movement. Likewise, the apparatus can be opened by a pivoting movement of the pressing device into one direction, and can be applied to the body and can be closed by a pivoting movement into the opposite direction. Therein, the pivoting movement does not necessarily have to performed at a predefined angle, although this would be possible. Instead, the proportion of the pivoting movement is preferably chosen depending on the body part, which is sandwiched by the movement. The pivoting movement can be driven electromechanically or hydraulically-However, it is preferred that the pressing device comprises a lever, which can be manipulated by hand for pressing the pressing device against the body part.

The application head and the pressing device can be moved with respect to each other along a sliding axle. On one hand, the movement along the sliding axle can be applied for sandwiching the body part to be treated between the pressing device and the application head with a linear movement only. On the other hand, the movement along the sliding axle can also be combined with a folding or a pivoting movement of the pressing device.

The movement of the application head and the pressing device along the sliding axle is lockable, and the body part can be sandwiched between the pressing device and the application head by the pivoting movement. Likewise, a coarse adjustment of the distance between the pressing device and the application head can be performed with the movement along the sliding axle, depending for example on the specific constitution of the skin and the anderlying tissue or the region to be treated. Therewith, the movement along the sliding axle can be locked for example with a screw or a snap. Subsequently, the specific body part to be treated can be pressed against the application head by a pivoting movement of the pressing device.

In one embodiment according to the invention, the shockwaves are generated within a water volume, which is bounded by a membrane, wherein the membrane is designed as a body contact surface of the application head. Shockwaves generated in a water volume, for example piezoelectrically, electromechanically, electromagnetically or electrohydraulically, are characterized by a rapid rise time, a high amplitude and a short pulse duration. As a result of the rapid interaction between pressure and shear loads, cavitation occurs, wherein the collapse of the gas bubbles leads to fast flows or jet streams, which contribute to the treatment effect on the tissue. Generating the shock waves in water is further advantageous, as the acoustic properties of water are similar to those of the tissue, so that coupling losses due to an impedance change are low- Besides water, also other liquids with an impedance (density multiplied by sound velocity) close to the impedance of the tissue could be applied, even though, water is preferred.

Preferably, the shockwaves generated within the water volume are generated by an electromagnetic generator having a coil adapted for a pulsed excitation, preferably a cylindricaf coil. The coil generates the shockwaves electromechanically, wherein current pulses of the generator induce the pulsed excitation of the coil. Current pulses with a rapid rise time can for example be obtained by discharging a capacitor.

In another embodiment according to the invention, the application head is an impact body, and a striking member strikes against the impact body to generate the shockwaves. A shock wave generation would also be possible with the striking member striking directly against the body part to be treated, and it would further be possible that the impact body is excited without the striking member, for example directly with an air pressure pulse. However, it is preferred that the striking member is accelerated onto the impact body, wherein the movement of the striking member can for example follow a linear or a circular line. In case of the circular line, the striking member can for example be driven electromechanically by an electric motor. In case of the linear line, the striking member can be moved repetitively back and forth to generate several shockwaves. The shockwaves generated by such a mechanical impact have a slower rise time, a lower amplitude and a longer pulse duration than the above-mentioned shockwaves generated within a water volume. Therefore, the interaction of the shockwave with the tissue is different and the type of shockwave will be chosen according to diagnosis and pathology as well as to the specific targets of the therapy.

Advantageously, the striking member is driven pneumatically. A shock wave could also be generated by applying a pneumatic pressure pulse directly to the body part to be treated, as well as by applying the pressure pulse directly to the impact body. However, it is preferred that the pneumatic pressure pulse can move the striking member along a linear line towards the impact body to generate the shockwave by an impact at the end of this movement. Repetitively moving the striking member back and forth generates repetitive impacts and therewith successive shock waves. A shock wave apparatus of that kind has for example been described in DE 90 2006 062 356 B3.

In one embodiment, the application head has focusing means for focusing the shockwaves into the body part. An electromagnetic shock wave generation in water volume for example can generate radial or cylindrical waves. Therefore, additionally focusing those waves can, depending on the treatment purpose, be necessary. The waves can for example be focused into the body part with lenses or a reflector with a focusing geometry, e.g. a concave or cone-like shape. Reference is made to EP 0 369 177 B1 for a more detailed description of reflector geometries.

The invention relates also to a use of a pressing device for mechanically connecting it to a handheld apparatus for treating the human or animal body by shockwaves, in order to create an apparatus according to one of the preceding claims.

In the following, the invention is described in detail with reference to an exemplary embodiment
Figure 1 shows a cross-sectional view of a body part being sandwiched between an application head and a pressing device.
Figure 2 shows the setup of figure 1 with pneumatical instead of electromagnetical shock wave generation.
Figure 3 presents the setup of figure 1 with a modified reflection plate.
Figure 4 shows a setup with two application heads.

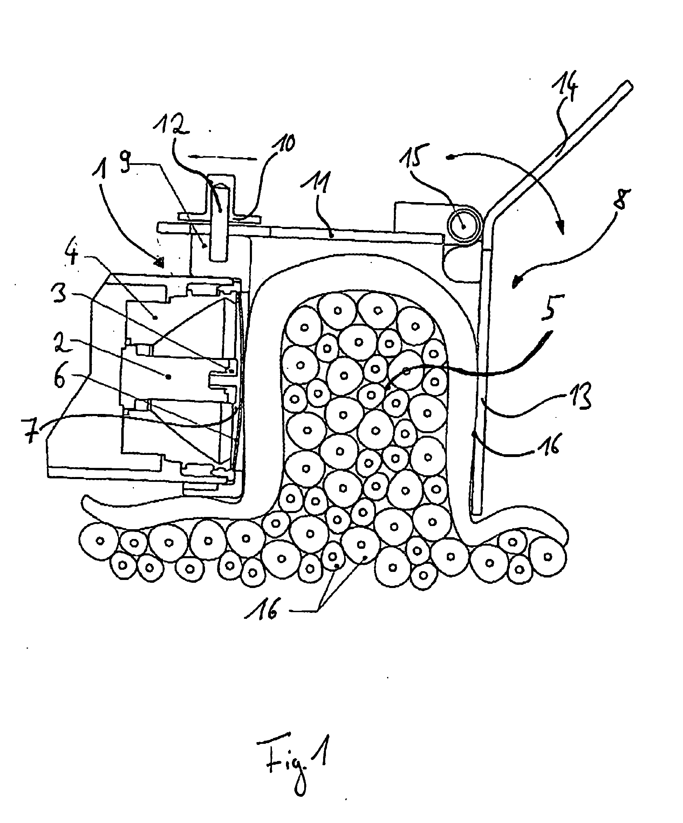

An application head 1 of a shockwave apparatus is shown in the left part of Fig. 1. Approximately cylindrical shock waves are generated electromechanically with a coil 2 having a coil cover 3 and are subsequently focused with a reflector 4 into the body part 5 to be treated. A membrane 6 is, in conjunction with the reflector 4 , enclosing a water volume 7, which allows a good coupling of the shock waves to the body part 5. Therewith, the membrane 6is designed as a body contact surface of the application head 1 and transmits the shock waves to the body part 5. The shock waves are propagating from the left to the right.

A pressing device 8 is mechanically connected to the application head 1 by a flange 9, which is mounted at the application head 1 with a screw or a snap mechanism. The flange 9 comprises a guiding device 10 for guiding an adjustment plate 11. The adjustment plate 11 is movable in the guiding device 10 in a direction basically parallel to the main propagation direction of the shockwaves, so that the pressing device 8 and the application head 1 can be moved with respect to each other along a sliding axle. Therewith, this movement can be locked with a fixation knob 12. A proximal end of the adjustment plate 11 is connected to the application head 1 , and a reflection plate 13 with a lever 14 is hinged 15 at a distal of the adjustment plate 11. Therewith, the reflection plate 13 acts as pressing device 8 for pressing the body part 5 to be treated against the application head 1 by a pivoting movement. A coarse adjustment, depending for example on the specific constitution of the skin and the underlying tissue with the cells 16 to be treated, can be performed prior to the pivoting movement by locking the movement along the sliding axle. Subsequently, the lever 14 can be manipulated by hand to perform the pivoting movement of the reflection plate 13 and to sandwich the body part 5 to be treated therewith. The reflection plate 13 can have a surface with a concave shape, which means a negative curvature, with respect to the application head 1.

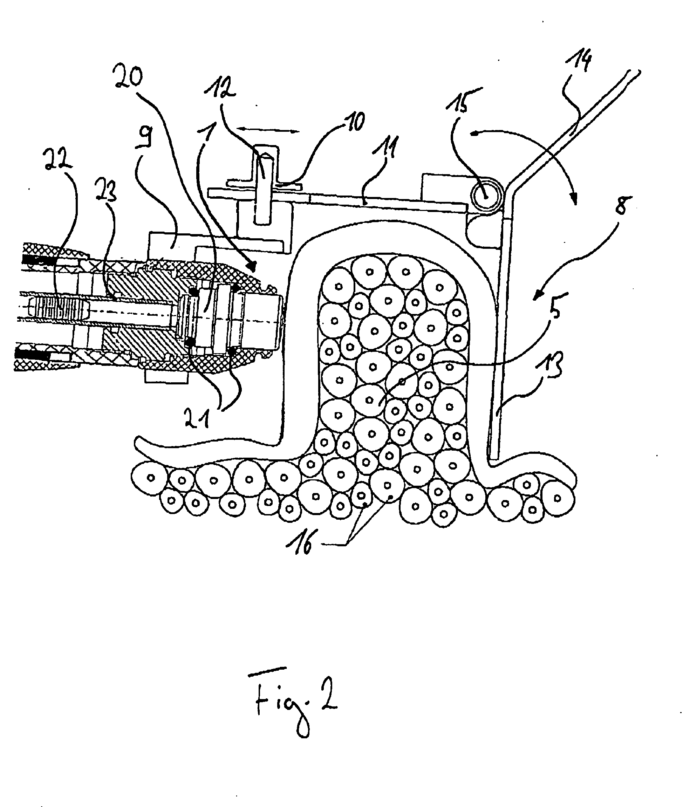

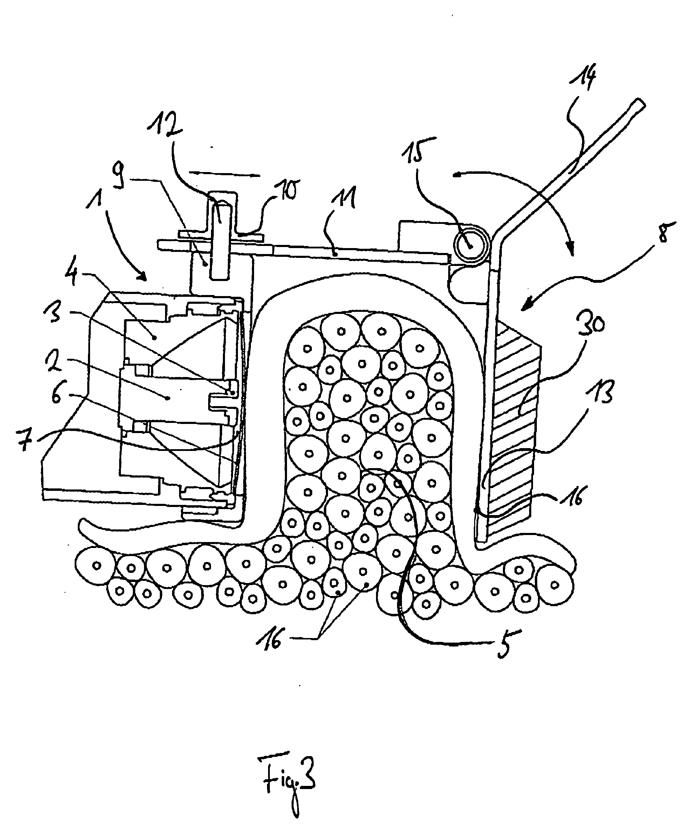

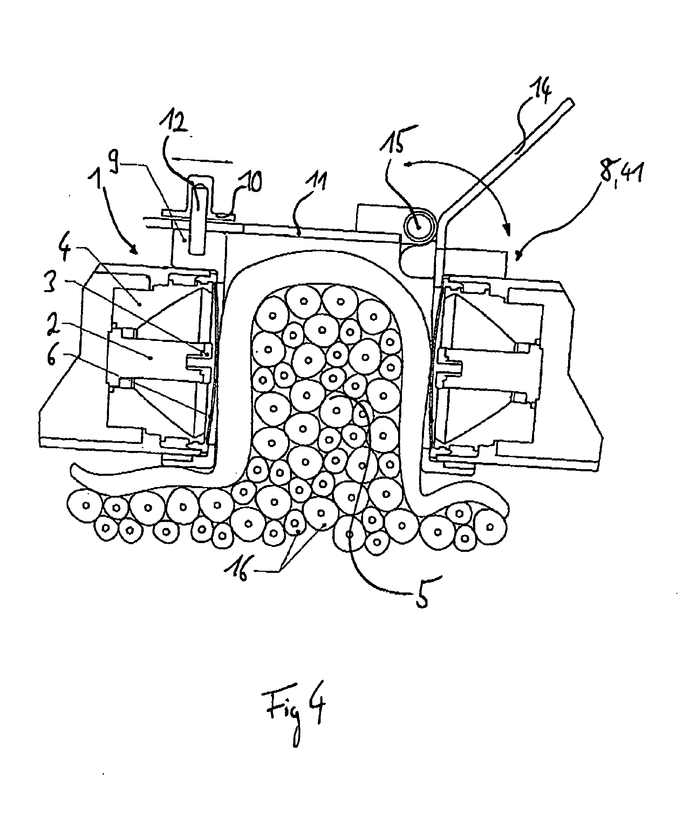

The pressing device 8 shown in figure 2 is of the same type as in figure 1, and identical reference numerals indicate parts having the same functionality (also in figures 3 and 4). The application head 1 has an impact body 20, which is elastically mounted in the application head 1 by two O-rings 21. A striking member 22 is accelerated in a guiding tube 23 onto the impact body 20 to create a shock wave by a mechanical impact, wherein the impact body 20 transmits the shock wave to the body part 5 to be treated. The striking member 22 can be moved repetitively back and forth along the linear line in the guiding tube 23 to generate shock waves. After a propagation of the shock waves through the body part 5, the reflection plate 13 reflects the shock waves partially to the body part 5 and likewise further increases the effectiveness of the treatment.

The embodiment shown in figure 3 has an electromagnetically driven application head 1 being identical to the one shown in figure 1. The pressing plate 13 is made of metal and has a smooth reflection surface 16. Additionally, a layer of foamed material 30 is, with respect to the main propagation direction, provided in sequence to the metal plate 13. Likewise, the shock waves are first partially reflected by the smooth surface 16 of the metal plate 13. However, even if a certain part of the shock waves propagates through the metal plate 13, the layer of foamed material 30 causes a change of impedance so that the energy of the shock waves is further reduced and body parts lying outside the sandwiched region are protected.

Figure 4 shows an embodiment with a first application head 1 and a second application head 41 acting as pressing device 8. The application heads 1/41 are both driven electromagnetically in this case and the parts connecting them mechanically have the same functionality as in figure 1.

It is also possible, to provide two different types of application heads 1/41, e. g. one being electromagnetically driven and the other being pneumatically driven. The second application head 41 is hinged 15 with the lever 14 at the distal end of the adjustment plate 11. Likewise, the second application head 41 acts as pressing device 8 for pressing the body part 5 against the first application head 1 by a pivoting movement

## Claims

1. A handheld apparatus for treating the human or animal body by shock waves,
said apparatus comprising:
a shock wave source adapted to produce said shock waves,
an application head (1) adapted to apply said shock waves to a part (5) of said body,
a pressing device (8) for pressing said body part (5) against said application head (1),
said pressing device (8) being mechanically connected to said apparatus by connecting means,
wherein said connecting means allow a movement of said pressing device (8) and said application head (1) towards each other,
so that said body part (5) can be sandwiched between said pressing device (8) and said application head (1) as a result of said movement towards each other,
wherein said application head (1) and said pressing device (8) can be moved with respect to each other along a sliding axle and said pressing device is hinged (15) for a pivoting movement, and
said movement of said application head (1) and said pressing device (8) along said sliding axle is lockable,
wherein said apparatus can be opened by said pivoting movement of said pressing device (8) into one direction and can be closed by said pivoting movement of said pressing device (8) into the opposite direction,
so that said body part (5) can be sandwiched between said pressing device (8) and said application head (1) by said pivoting movement into the opposite direction,
**characterized in that**
said pressing device (8) comprises a pressing plate (13) manipulable by hand to perform the pivoting movement and extending transversally to a main propagation direction of said shock waves when closed by said pivoting movement into the opposite direction.

2. The apparatus according to claim 1, wherein said pressing device (8) has a pressing surface (16) with a concave shape.

3. The apparatus according to one of the preceding claims, wherein said pressing device (8) has a pressing surface (16), which is formed of metal.

4. The apparatus according to one of the preceding claims, wherein said pressing device comprises, in a main propagation direction of said shock waves, a layer of metal and a layer of foamed material (30) in sequence.

5. The apparatus according to one of the preceding claims, wherein said shock waves are generated within a water volume (7), said water volume being bounded by a membrane (6), which is designed as a body contact surface of said application head (1).

6. The apparatus according to claim 5, wherein said shock waves are generated by an electromagnetic generator having a coil (2) adapted for pulsed excitation.

7. The apparatus according to one of claims 1 to 5, wherein said application head (1) has an impact body (20), and a striking member (22) strikes against said impact body (20) to generate said shock waves.

8. The apparatus according to claim 7, wherein said striking member (22) is driven pneumatically.

9. The apparatus according to one of the preceding claims, wherein said application head (1) has focusing means (4) for focusing said shock waves into said body part.

10. Use of a pressing device (8) for mechanically connecting it to a handheld apparatus, which is provided for treating the human or animal body by shock waves, in order to create an apparatus according to one of the preceding claim, the use consisting of mechanically connecting said pressing plate (13) to said apparatus by said connecting means.

## Patentansprüche

1. Handgerät zur Behandlung des menschlichen oder tierischen Körpers mit Druckwellen,
welches Gerät aufweist:
eine Druckwellenquelle, die dazu ausgelegt ist, die Druckwellen zu erzeugen,
einen Applikatorkopf (1), der dazu ausgelegt ist, die Druckwellen einem Körperteil (5) des Körpers zuzuführen,
eine Andrückeinheit (8) zum Andrücken des Körperteils (5) gegen den Applikatorkopf (1),
welche Andrückeinheit (8) mit dem Gerät über ein Verbindungsmittel mechanisch verbunden ist,
welches Verbindungsmittel ein Aufeinanderzubewegen von Andrückeinheit (8) und Applikatorkopf (1) ermöglicht,
sodass der Körperteil (5) zwischen der Andrückeinheit (8) und dem Applikatorkopf (1) infolge deren Relativbewegung aufeinander zu zusammengedrückt werden kann,
wobei der Applikatorkopf (1) und die Andrückeinheit (8) entlang einer Schiebeachse relativ zueinander verschoben werden können und die Andrückeinheit für eine Schwenkbewegung gelenkig gelagert (15) ist, und
die Relativbewegbarkeit von Applikatorkopf (1) und Andrückeinheit (8) entlang der Schiebeachse arretierbar ist,
wobei das Gerät durch die Schwenkbewegung der Andrückeinheit (8) in eine Richtung geöffnet werden kann und durch die Schwenkbewegung der Andrückeinheit (8) in die entgegengesetzte Richtung geschlossen werden kann,
sodass der Körperteil (5) durch die Schwenkbewegung in die entgegengesetzte Richtung zwischen der Andrückeinheit (8) und dem Applikatorkopf (1) zusammengedrückt werden kann,
**dadurch gekennzeichnet, dass**
die Andrückeinheit (8) ein Andrückschild (13) aufweist, welches zum Vollführen der Schwenkbewegung von Hand bedienbar ist und sich in dem durch die Schwenkbewegung in die entgegengesetzte Richtung geschlossenen Zustand quer zu einer Hauptausbreitungsrichtung der Druckwellen erstreckt.

2. Gerät nach Anspruch 1, bei welchem die Andrückeinheit (8) eine Andrückoberfläche (16) mit einer konkaven Form hat.

3. Gerät nach einem der vorstehenden Ansprüche, bei welchem die Andrückeinheit (8) eine Andrückoberfläche (16) hat, die aus Metall gebildet ist.

4. Gerät nach einem der vorstehenden Ansprüche, bei welchem die Andrückeinheit hinsichtlich einer Hauptausbreitungsrichtung der Druckwellen aufeinanderfolgend eine Lage Metall und eine Lage geschäumten Materials (30) aufweist.

5. Gerät nach einem der vorstehenden Ansprüche, bei welchem die Druckwellen in einem Wasservolumen (7) erzeugt werden, welches Wasservolumen durch eine Membran (6) begrenzt wird, welche Membran als Körperkontaktfläche des Applikatorkopfs (1) ausgelegt ist.

6. Gerät nach Anspruch 5, bei welchem die Druckwellen mit einem elektromagnetischen Erzeuger erzeugt werden, welcher eine für eine gepulste Anregung ausgelegte Spule (2) aufweist.

7. Gerät nach einem der Ansprüche 1 bis 5, bei welchem der Applikatorkopf (1) einen Prallkörper (20) aufweist und ein Schlagteil (22) gegen den Prallkörper (20) schlägt, um die Druckwellen zu erzeugen.

8. Gerät nach Anspruch 7, bei welchem das Schlagteil (22) pneumatisch angetrieben ist.

9. Gerät nach einem der vorstehenden Ansprüche, bei welchem der Applikatorkopf (1) Fokussiermittel (4) zum Fokussieren der Druckwelle in den Körperteil aufweist.

10. Verwendung einer Andrückeinheit (8), um sie mechanisch mit einem Handgerät, welches zur Behandlung des menschlichen oder tierischen Körpers mit Druckwellen vorgesehen ist, zu verbinden, um ein Gerät nach einem der vorstehenden Ansprüche zu schaffen, wobei die Verwendung darin besteht, das Andrückschild (13) über das Verbindungsmittel mechanisch mit dem Gerät zu verbinden.

## Revendications

1. Appareil a main destiné au traitement d'un corps humain ou animal au moyen d'ondes de choc,
ledit appareil comprenant :
une source d'ondes de choc adaptée pour produire lesdites ondes de choc,
une tête d'application (1) adaptée pour appliquer lesdites ondes de choc à une partie (5) dudit corps,
un dispositif de pressage (8) destiné à presser ladite partie du corps (5) contre ladite tête d'application (1),
ledit dispositif de pressage (8) étant relié mécaniquement audit appareil par un moyen de liaison,
dans lequel ledit moyen de liaison permet un déplacement mutuel dudit dispositif de pressage (8) et de ladite tête d'application (1),
de telle manière que ladite partie du corps (5) peut être prise en sandwich entre ledit dispositif de pressage (8) et ladite tête d'application (1) sous l'effet dudit déplacement mutuel,
dans lequel ladite tète d'application (1) et ledit dispositif de pressage (8) peuvent être déplacés l'un par rapport à l'autre sur un axe de coulissement et ledit dispositif de pressage est articulé (15) pour permettre un déplacement de pivotement, et
ledit déplacement de ladite tête d'application (1) et dudit dispositif de pressage (8) sur ledit axe de coulissement est blocable,
dans lequel ledit appareil peut être ouvert sous l'effet dudit déplacement de pivotement dudit dispositif de pressage (8) dans une direction et peut être fermé sous l'effet dudit déplacement de pivotement dudit dispositif de pressage (8) dans la direction inverse,
de telle manière que ladite partie du corps (5) peut être prise en sandwich entre ledit dispositif de pressage (8) et ladite tête d'application (1) sous l'effet dudit déplacement de pivotement dans la direction inverse,
**caractérisé en ce que**
ledit dispositif de pressage (8) comprend une plaque de pressage (13) pouvant être manipulée à la main dans le but d'effectuer le déplacement de pivotement et s'étendant de manière transversale à une direction principale de propagation desdites ondes de choc lorsqu'elle est fermée sous l'effet dudit déplacement de pivotement dans la direction inverse.

2. Appareil selon la revendication 1, dans lequel ledit dispositif de pressage (8) présente une surface de pressage (16) de forme concave.

3. Appareil selon l'une des revendications précédentes, dans lequel ledit dispositif de pressage (8) présente une surface de pressage (16) constituée de métal.

4. Appareil selon l'une des revendications précédentes, dans lequel ledit dispositif de pressage comprend, dans une direction principale de propagation desdites ondes de choc, une couche de métal et une couche de matière expansée (30) disposées en séquence.

5. Appareil selon l'une des revendications précédentes, dans lequel lesdites ondes de choc sont générées à l'intérieur d'un volume d'eau (7), ledit volume d'eau étant délimité par une membrane (6) qui est conçue comme une surface de contact avec le corps de ladite tête d'application (1).

6. Appareil selon la revendication 5 dans lequel lesdites ondes de choc sont générées par un générateur électromagnétique présentant une bobine (2) adaptée pour une excitation pulsée.

7. Appareil selon l'une des revendications 1 à 5, dans lequel ladite tête d'application (1) présente un corps d'impact (20), et un élément de frappe (22) frappe contre ledit corps d'impact (20) pour générer lesdites ondes de choc.

8. Appareil selon la revendication 7, dans lequel ledit élément de frappe (22) est entraîné par voie pneumatique.

9. Appareil selon l'une des revendications précédentes, dans lequel ladite tête d'application (1) présente un moyen de focalisation (4) destiné à focaliser lesdites ondes de choc sur ladite partie du corps (5).

10. Utilisation d'un dispositif de pressage (8) visant à le relier de manière mécanique à un appareil portatif qui est prévu pour traiter un corps humain ou animal au moyen d'ondes de choc, dans le but de créer un appareil selon l'une des revendications précédentes, ladite utilisation consistant à relier de manière mécanique ladite plaque de pressage (13) audit appareil par ledit moyen de liaison.
